# EUROPÄISCHE PATENTSCHRIFT

(11) **EP 0 343 501 B1**
(45) Veröffentlichungstag und Bekanntmachung des Hinweises auf die Patenterteilung: **30.06.1993**
(21) Anmeldenummer: 89108901.3
(22) Anmeldetag: 18.05.1989
(51) Int. Cl.: A61M 5/14

(54) **Mehrfachinfusionssystem**
Multiple infusion system
Système de perfusion multiple

(30) Priorität: 21.05.1988 DE 3817411
(43) Veröffentlichungstag der Anmeldung: 29.11.1989
(73) Patentinhaber: Fresenius AG, 61350 Bad Homburg (DE)
(72) Erfinder: Polaschegg, Hans Dietrich, Dr., D-6370 Oberursel 4 (DE)
(74) Vertreter: Fuchs Mehler Weiss

(56) Entgegenhaltungen:
- EP-A- 0 040 592
- EP-A- 0 181 691
- EP-A- 0 189 491
- FR-A- 2 314 753
- FR-A- 2 321 904
- FR-A- 2 338 709
- US-A- 3 648 694
- US-A- 4 177 808

## Beschreibung

Die Erfindung betrifft eine Vorrichtung zur dosierten, gleichzeitigen Infusion von mehreren Infusionslösungen oder Medikamenten mit mindestens einer Pumpe mit Förderleitungen, die von den die Infusions- oder Medikamentlösungen aufweisenden Behältern abgehen und über ein Übergangsstück mit einer gemeinsamen Patientenleitung verbunden sind, mit Ventilen, die in jeder Förderleitung vorgesehen sind, mit einem den Durchfluß detektierenden Durchflußelement und mit einer Steuereinheit, die mit den Ventilen, der Pumpe und dem Durchflußelement signalmäßig verbunden ist.

Eine derartige Vorrichtung ist aus der DE-C-3 329 977 (EP-A-0 189 491) bekannt.

Die moderne Intensivtherapie benutzt in zunehmendem Maße Infusionspumpen zur künstlichen, parenteralen Ernährung, zur kontinuierlichen bzw. genau dosierten Applikation von Medikamenten und neuerdings auch zur geregelten Zufuhr hochwirksamer Medikamente in Abhängigkeit von physiologischen Parametern.

In der Regel werden drei bis vier unterschiedliche Medikamente bzw. Infusionslösungen mit Hilfe mehrerer Pumpen bzw. voneinander unabhängigen Regeleinrichtungen über einen Zugang zum Blutkreislauf des Patienten appliziert, der gewöhnlich aus einem zentralvenösen Katheter besteht. In Ausnahmefällen können bis zu zwölf Pumpen an einem Patientenzugang angeschlossen sein.

Gewöhnlich benutzt man dazu Pumpen mit den dazugehörigen Einmalschlauchartikeln, die stromab der Pumpe über Drei-Wegehähne mit einer Sammelleitung, die zum Katheter führt, verbunden sind. An der gleichen Patientenleitung sind üblicherweise zusätzlich ebenfalls über Drei-Wegehähne noch sogenannte Schwerkraftinfusionsgeräte und Einrichtungen zur Messung des zentralvenösen Druckes angeschlossen.

Derartige Anordnungen weisen folgende Nachteile auf:
1. Der individuelle Aufbau ist in der Regel unübersichtlich, so daß die Gefahr von Verwechslungen gegeben ist.
2. Das individuelle Zusammenstecken von Infusionsleitungen, Drei-Wegehähnen und dergleichen führt zur Gefahr der Kontamination.
3. Bei einer Kombination von Pumpen und Schwerkraftinfusionsgeräten kommt es bei Verlegung des Patientenzuganges zu einer Förderung von Infusionslösung aus den Pumpen in das Schwerkraftinfusionssystem, da die Druckerkennungssysteme der Pumpen, falls überhaupt welche vorhanden sind, bei einem durch die Schwerkraft hervorgerufenen Druck von maximal 0,2 bar nicht ansprechen. Der Infusionsstop bleibt daher unerkannt bzw. wird erst mit Hilfe des Patientenmonitoring indirekt erkannt.
4. Wird die Infusionsleitung stromab der Pumpen unterbrochen (z.B. durch einen versehentlich geschlossenen Drei-Wegehahn), so pumpt die Pumpe bzw. arbeiten die Pumpen so lange weiter, bis auf Grund eines zu hohen Druckes in der Leitung das Druckerkennungssystem einer Pumpe anspricht bzw. die Pumpe aufhört zu fördern.

Aufgrund der Verformbarkeit der Infusionsleitungen kommt es zur Ausbildung eines Speichervolumens, das sich beim Öffnen des Drei-Wegehahnes bzw. nach der Beseitigung der Unterbrechnung schlagartig in den Patienten entleeren kann.

Das irrtümliche Verschließen eines Dreiwegehahnes kommt relativ häufig vor, da z.B. zur Probenentnahme, aber auch zur Messung des zentralvenösen Druckes (CVD-Messung) die Verbindungshähne zu den Pumpen kurzzeitig geschlossen werden müssen.

Es gibt zwar gewisse Verhaltensmaßregeln und Vorschriften, nach denen die vorgenannten Gefährdungen vermieden werden sollen, jedoch verlangen diese ein großes Konzentrationsvermögen des Anwenders bzw. sie sind mit erhöhten Kosten verbunden. So gilt es beispielsweise nicht als fachgerecht, eine Pumpeninfusion mit einer Schwerkraftinfusion zu kombinieren. Dies führt wiederum dazu, daß selbst dort eine Infusionspumpe eingesetzt werden muß, wo dies von der Genauigkeitsanforderung an die Förderung des Medikamentes bzw. der Infusionslösung nicht gerechtfertigt ist, was zu erhöhten Kosten führt.

Um einen Teil der genannten Nachteile zu vermeiden, sind sogenannte Mehrfachinfusionssysteme entwickelt worden. Aus o.g. DE-PS 33 29 977 ist eine Vorrichtung zur dosierten Infusion von Lösungen aus mehreren Infusionsbehältern bekannt. Mehrere Schwerkraftinfusionseinrichtungen mit Tropfenzähler sind über Ablaufleitungen, in die Klemmventile eingebaut sind, an einer Sammelleitung angeschlossen, die zum Patienten führt. In dieser Patientenleitung ist stromab zunächst ein Durchflußsensor und danach eine Förderpumpe angeschlossen. Alle Ventile und der Durchflußsensor sowie die Pumpe sind mit einer Steuereinheit verbunden.

Die vom Arzt zu wählende Infusionsrate für die einzelnen Lösungen werden in dem Steuergerät in proportionale Tropfenzahlen umgerechnet. Jedes Ventil bleibt solange geöffnet, bis die diesem Kanal zugeordnete Tropfenzahl erreicht ist. Danach wird das Ventil geschlossen und das Ventil des nächsten Kanals geöffnet. Es werden jeweils bestimmte Tropfenzahlen ermittelt und der Sammelleitung zugeführt, bis eine Umschaltung erfolgt. Diese Vorrichtung hat eine Reihe von Nachteilen. Die Infusion erfolgt sequentiell, d.h. die Förderung der einzelnen Lösungen erfolgt nicht kontinuierlich. Außerdem ist die Förderung eines hoch wirksamen Medikamentes mit geringerer Rate und gleichzeitig beispielsweise einer parenteralen Nährlösung mit hoher Rate nicht möglich. Ein weiterer Nachteil besteht darin, daß die einzige Pumpe mit konstanter Förderrate arbeitet und ein Defekt in der Pumpe zu einer unkontrollierten Infusion führt.

Ein einer Pumpe nachgeschaltetes, den Durchfluß detektierendes Durchflußelement ist bereits bekannt (FR-A-2 338 709), jedoch ist dabei die Überwachung des von mehreren Pumpen zusätzlich mindestens einer Schwerkraft-Infusionseinrichtung zugeführten Flüssigkeitsstromes nicht vorgesehen und auch nicht möglich.

Aufgabe der vorliegenden Erfindung ist es, die genannten Nachteile zu vermeiden und eine Vorrichtung zur dosierten Infusion anzugeben, die die gleichzeitige Förderung von hoch wirksamen Medikamenten mit kleinen Infusionsraten bei hoher Genauigkeit, von hohen Infusionsraten bei mittler Genauigkeit sowie von hohen Infusionsraten mit mäßiger Genauigkeit erlaubt, wobei gegenüber herkömmlichen Einrichtungen nicht nur eine einfachere und sicherere Anwendung, sondern auch eine Kostenersparnis erzielt wird.

Diese Aufgabe wird durch eine Vorrichtung gemäß den kennzeichnenden Merkmalen von Anspruch 1 gelöst.

Bei der erfindungsgemäßen Mehrfachinfusionsvorrichtung können mehrere Pumpen und Schwerkrafteinrichtungen miteinander kombiniert werden. So können Spritzeninfusionspumpen, die eine hohe Genauigkeit bei kleinen Infusionsraten gewährleisten, mit linearperistaltischen Infusionspumpen, die eine mittlere Genauigkeit bei hohen Infusionsraten gewährleisten, miteinander kombiniert sein. Für hohe Infusionsraten mit mäßiger Genauigkeit sind Schwerkraftinfusionseinrichtungen vorgesehen. Sowohl die Pumpen als auch die Schwerkraftinfusionseinrichtungen sind stromab an Förderleitungen angeschlossen, die in eine gemeinsame Patientenleitung münden. In diesen Förderleitungen sind jeweils Klemmventile eingebaut, die an eine Recheneinheit angeschlossen sind, die auch eine Steuereinheit aufweist. Die Recheneinheit ist gleichzeitig an die Pumpen angeschlossen, so daß die vorgegebenen Infusionsraten, die in die Recheneinheit eingegeben wurden, an die Pumpen weitergegeben werden können. Die Recheneinheit kann ferner mit einem Strichcodeleser ausgestattet sein, der es erlaubt, Infusionsbehältnisse, die ihrerseits mit einem Strichcode gekennzeichnet sind, datenmäßig zu erfassen. Die Recheneinheit verfügt dann sowohl über Informationen zur Zusammensetzung der angeschlossenen Infusionslösung als auch zur Rate, mit der diese appliziert werden. Damit kann die Recheneinheit sowohl die Gesamtmenge der zu jedem Zeitpunkt dem Patienten applizierten Flüssigkeitsmenge als auch Energiemenge und spezifische Stoffmenge (Natrium, Kalium, andere Elektrolyte, Aminosäuren, Fette, Glucose und dergleichen) errechnen und auf einem Bildschirm anzeigen oder auf einem Drucker ausgeben. Anstelle eines Barcodelesers können auch andere Lesevorrichtungen, die eine automatische Erfassung erlauben, verwendet werden. Sind solche Einrichtungen auf dem Infusionslösungsbehälter nicht vorgesehen, so kann auch eine manuelle Eingabe erfolgen. In der Patientenleitung ist ebenfalls ein Ventil angeordnet.

Wichtig bei der erfindungsgemäßen Vorrichtung ist es, daß das Durchflußelement stromab der Pumpen angeordnet ist und daß die Steuereinheit die Vorrichtung bei Erkennen eines Fluidstopps durch das Durchflußelement durch Schließen der Ventile und Abschalten der Pumpe desaktiviert.

Vorzugsweise ist das Durchflußelement in der Patientenleitung angeordnet.

Das Durchflußelement in der gemeinsamen Patientenleitung stromab der Pumpen bzw. stromab der Vereinigung aller Infusionsleitungen hat den Zweck, die Unterbrechung der Infusion, zum Beispiel durch eine Blockade des Katheters, zu erkennen. Ein Verschluß des Katheters, sei es durch Abknicken der Zuleitung oder aus anderen Gründen, kann weitreichende und schwerwiegende Folgen für den Patienten haben. Zunächst wird der Patient nicht mit den in den Infusionslösungen vorhandenen Medikamenten versorgt. Da darunter Medikamente mit Halbwertszeiten von wenigen Minuten sein können (zum Beispiel Natriumnitroprussid, das für die Blutdruckregelung verwendet wird), kann dies schon nach kurzer Zeit zu einem gefährlichen Anstieg des Blutdrucks beim Patienten führen. Es kann aber auch durch die Pumpwirkung zu einem Anstieg des Druckes in den Infusionsleitungen vor der Verschlußstelle kommen. Da diese elastisch sind, wird eine gewisse Menge Infusionslösung gespeichert. Kommt es schließlich zu einem Infusionsstopp, weil ein üblicherweise in Infusionspumpen vorhandener Druckdetektor diesen Druckanstieg detektiert und wird die Verschlußstelle danach beseitigt, so wird das gespeicherte Volumen schlagartig in den Patienten infundiert, wodurch es zur Mehrinfusion kommt.

Das Durchflußelement stromab der Pumpe kann eine Unterbrechung des Durchflusses erkennen, ehe noch ein Druckaufbau erfolgt ist. Zwar befindet sich auch stromab des Durchflußelements noch ein Schlauchstück mit gewisser Elastizität, jedoch ist das darin speicherbare Volumen wesentlich geringer als im gesamten System. Dazu kommt noch, daß im Falle des Verschlusses und des Druckaufbaus ein wesentlich größerer Teil des von den Pumpen geförderten Flusses in dem größeren Volumen des Schlauchsystems oberhalb, d.h. stromauf des Durchflußelements, gespeichert wird und damit der Fluß durch das Durchflußelement in das stromab des Durchflußelements gelegene Schlauchstück stark zurückgeht. Obwohl der Fluß in diesem Moment nicht auf Null geht, so wird er doch um einen großen Wert, mehr als 50 %, abnehmen. Diese Änderung ist leicht detektierbar. Als Folge einer solchen Detektion werden nicht nur die Ventile gestoppt, sondern auch die Pumpen. Die Pumpen dürfen in keinem Fall gegen die Ventile arbeiten, es sei denn zu irgendwelchen Test- und Prüfzwecken, da ansonsten vor den Ventilen ein Speichervolumen aufgebaut wird.

Vorzugsweise besteht das Durchflußelement aus einem Tropfendetektor. Zusätzlich kann an diese Patientenleitung noch eine CVD-Meßeinrichtung angeschlossen sein.

Gemäß einer weiteren Ausführungsform kann in der Patientenleitung zusätzlich noch ein Luftdetektor angeordnet sein, der ebenfalls an die Recheneinheit angeschlossen ist.

Vorteilhafterweise werden die genannten Schlauchklemmventile mechanisch betätigt und elektrisch überwacht oder aber durch die Recheneinheit elektrisch betätigt und redundant überwacht.

Vorzugsweise sind die Klemmventile und die Sensoren in einem sogenannten Sensor- und Ventilblock zusammengefaßt.

Ist mindestens eine Schwerkraftinfusionseinrichtung angeschlossen, so kann das Durchflußelement anstatt in der Patientenleitung auch in der entsprechenden Verbindungsleitung zur Schwerkraftinfusionseinrichtung angeordnet sein. Da die Schwerkraftinfusionseinrichtungen meist einen Tropfenregler mit manueller oder automatisch arbeitender Klemme aufweisen, so kann dieser die Aufgabe des Durchflußelements übernehmen. Das Signal von dem dazugehörigen Tropfensensor wird dazu benutzt, einen Verschluß der Patientenleitung zu erkennen.

Die Erkennung ist wie folgt möglich:
Im Normalfall fördern die Pumpen und parallel dazu die Schwerkraftinfusionseinrichtung Infusionslösung zum Patienten. Wird die Patientenleitung zum Beispiel durch eine Blockade des Katheters unterbrochen, so wird aufgrund der Pumpwirkung der Druck im System erhöht, wodurch die Tropfrate im schwerkraftbetriebenen System zurückgeht und schließlich zu Null wird: Die Pumpen fördern rückwärts in das Schwerkraftinfusionssystem hinein. Dies ist ein an sich gefährlicher Zustand, da dadurch die Unterbrechung der Infusion nicht erkannt wird. In der erfindungsgemäßen Ausführung wird dieser Zustand jedoch von dem Tropfensensor erkannt, so daß die Vorrichtung desaktiviert werden kann.

Die erfindungsgemäße Vorrichtung erlaubt die gefahrlose Kombination der Pumpen mit den angeschlossenen Schwerkraftinfusionseinrichtungen, da eine Unterbrechung des Flusses zum Patienten durch das Durchflußelement erkannt wird. Die von der Recheneinheit elektrisch gesteuerten bzw. überwachten Ventile verhindern, daß ein Ventil unabsichtlich geschlossen bleibt, obwohl die Pumpen in Betrieb genommen wurden. Falls eine CVD-Meßeinrichtung an die Patientenleitung angeschlossen ist, ist zusätzlich eine automatische Durchführung dieser Messung möglich. Der zentralvenöse Druck kann mit Hilfe einer manuell oder automatisch abschließbaren Wassersäule erfaßt werden. Eine Einrichtung zur automatischen Erfassung der Wassersäule kann vorteilhafterweise als Ultraschallniveausensor ausgeführt sein, wobei im Steigrohr eine ultraschallreflektierende Stelle als Referenzstrecke vorgesehen ist.

Bei dem Luftdetektor kann es sich um einen bekannten Ultraschalluftdetektor handeln. Dieser Luftdetektor ist vorteilhafterweise an das Ventil in der Patientenleitung angeschlossen, so daß dieses bei Ansprechen des Luftsensors geschlossen wird.

Zur Entlüftung der Leitungen ist an die Patientenleitung vorteilhafterweise eine Entlüftungseinrichtung angeschlossen. Zunächst werden alle Ventile in den Förderleitungen geschlossen, was durch die Rechner- und Steuereinheit erfolgt. Wenn die Patientenleitung mit einer Kombination aus Hydrophobfilter und Rückschlagventil ausgestattet ist, kann auch das Entlüftungsventil der Entlüftungseinrichtung geschlossen bleiben. Anderenfalls wird dieses durch die Steuereinheit geöffnet.

Anschließend wird das Ventil in der ersten Förderleitung geöffnet und die in diesem Zweig befindliche Pumpe in Betrieb genommen, die solange mit hoher Rate fördert, bis der Luftsensor Luftfreiheit signalisiert. Dann werden dieses Ventil in der Förderleitung sowie das Entlüftungsventil geschlossen und die Pumpe gestoppt.

Im Anschluß daran werden das Ventil in der Patientenleitung sowie das Ventil in der zweiten Förderleitung geöffnet und die Pumpe in diesem Zweig in Betrieb genommen. Diese fördert zunächst Luft, mit der die vom vorhergehenden Füllvorgang vorhandene Restmenge Flüssigkeit in der Patientenleitung aus dieser herausgepumpt und verworfen wird. Auf diese Weise erfolgt gleichzeitig eine Spülung der Schlauchleitung. Sobald der Luftsensors anspricht, wird das Ventil in der Patientenleitung geschlossen und das Entlüftungsventil geöffnet. Die Förderung der Pumpe wird fortgesetzt, bis der Luftsensor Luftfreiheit anzeigt. Die Pumpe wird angehalten und das Ventil in dieser Förderleitung wieder geschlossen. Auf diese Weise werden auch die übrigen Förderleitungen entlüftet.

Nach Abschluß dieser Maßnahme wird die Patientenleitung an den Patienten angeschlossen und die Infusion kann beginnen.

Wird gemäß einer weiteren Ausführungsform eine CVD-Meßeinrichtung an die Patientenleitung angeschlossen, so wird diese dadurch in Betrieb genommen, daß das Ventil in der Zuleitung zur Patientenleitung sowie das Ventil in einer Förderleitung geöffnet wird und alle anderen Ventile geschlossen bleiben. Die an diese Förderleitung angeschlossene Pumpe wird in Gang gesetzt, die dann solange Flüssigkeit fördert, bis die CVD-Meßeinrichtung entlüftet ist oder anspricht. Die Schwerkraftinfusionseinrichtung wird auf analoge Weise in Betrieb genommen. Hierzu werden das Ventil in der Förderleitung der Schwerkraftinfusionseinrichtung und das Entlüftungsventil geöffnet. Durch die Schwerkraft dringt Infusionslösung in die Förderleitung ein und verdrängt daraus die Luft. Sobald der Luftsensor Luftfreiheit anzeigt, werden diese Ventile, d.h. das Ventil in der Förderleitung zur Infusionseinrichtung und das Entlüftungsventil wieder geschlossen.

Werden eine Schwerkraftinfusionseinrichtung mit einem Tropfensensor an die Förderleitung angeschlossen und der erwähnte Tropfensensor mit der Rechner- und Steuereinheit signalmäßig verbunden, so kann letztere in bekannter Weise das Ventil in der Förderleitung so öffnen und schließen, daß im Mittel eine vorgegebene Tropfenrate erreicht wird. Dies bedeutet, daß das System als Tropfeninfusionsregler arbeitet.

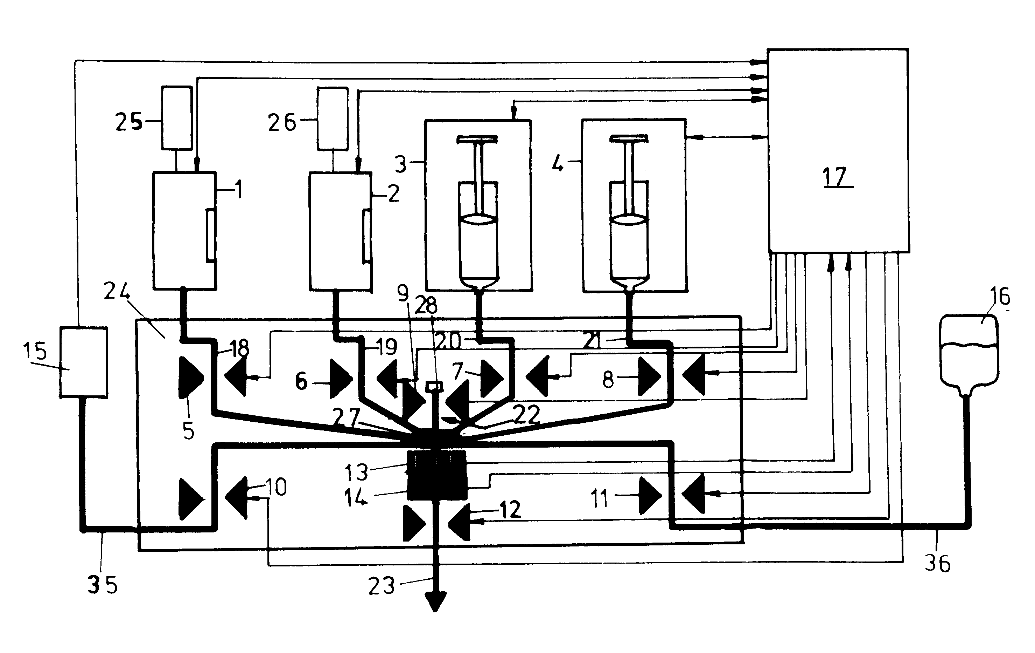

Nachfolgend wird eine beispielhafte Ausführungsform anhand der Zeichnung näher erläutert.

In der Figur ist eine Mehrfachinfusionseinrichtung dargestellt, die zwei linearperistaltische Infusionspumpen 1 und 2, zwei Spritzeninfusionspumpen 3 und 4, eine Schwerkraftinfusionseinrichtung 16 und eine CVD-Meßeinrichtung 15 aufweist. Die linearperistaltischen Infusionspumpen 1 und 2 sind jeweils an Infusionslösungsbehälter 25 und 26 angeschlossen. Jede Infusionspumpe ebenso wie die Schwerkraftinfusionseinrichtung ist an eine Förderleitung angeschlossen, die mit 18, 19, 20, 21 und 36 bezeichnet sind. In jeder Förderleitung befindet sich ein Klemmventil 5, 6, 7, 8, 11, die signalmäßig mit einer Rechen- und Steuereinheit 17 verbunden sind. Diese Rechen- und Steuereinheit ist gleichzeitig an die Infusionspumpen 1, 2, 3 und 4 angeschlossen. Die Förderrate dieser Infusionspumpen wird durch die Rechen- und Steuereinheit entsprechend einem vorbestimmten Programm geregelt.

Die Förderleitungen 18, 19, 20, 21 und 36 sowie die Zuleitung 35 zur CVD-Meßeinrichtung 15 sind über ein Übergangsstück 27 gemeinsam an die Patientenleitung 23 angeschlossen. In dieser Patientenleitung 23 sind ein Luftsensor 13, ein Durchflußelement 14 sowie ein weiteres Ventil 12 angeordnet. Der Luftsensor, der Durchflußsensor und dieses Ventil 12 sind ebenfalls an die Rechen- und Steuereinheit 17 angeschlossen.

An das Übergangsstück 27 ist zusätzlich noch eine Entlüftungseinrichtung 22 in Form eines Stutzens mit dem Ventil 9 angeschlossen, das ebenfalls mit der Rechen- und Steuereinheit 17 verbunden ist. Anstelle des Ventils 9 kann auch ein hydrophobes Filter 28, gegebenenfalls mit einem nicht gezeigten Rückschlagventil, die Entlüftungseinrichtung 22 gegenüber der Umgebung steril verschließen.

Diese Mehrfachinfusionseinrichtung besteht somit aus unterschiedlichen Infusionseinrichtungen, mit denen unterschiedliche Mengen an Infusionslösungen bzw. Medikamenten mit unterschiedlicher Genauigkeit appliziert werden können. Durch die Anordnung von Luftsensor 13 und Durchflußsensor 14 in der Patientenleitung kann auf die üblicherweise in den Förderleitungen angeordneten Druck- und Luftsensoren verzichtet werden. Diese erfindungsgemäße Anordnung gewährleistet ein hohes Maß an Sicherheit für den Patienten, da eine Unterbrechung des Flusses zum Patienten durch den Durchflußsensor 14 erkannt wird und über die Rechen- und Steuereinheit dann die entsprechenden Ventile in den Förderleitungen oder auch das Sicherheitsventil 12 sofort geschlossen werden kann. Darüber hinaus bietet die Mehrfachinfusionsvorrichtung die Möglichkeit, eine CVD-Meßeinrichtung 15 ebenfalls an die Patientenleitung 23 anzuschließen.

## Patentansprüche

1. Vorrichtung zur dosierten, gleichzeitigen Infusion von mehreren Infusionslösungen oder Medikamenten mit mindestens einer Pumpe (1 bis 4), mit Förderleitungen (18, 19, 20, 21, 36), die von den die Infusions- oder Medikamentenlösungen aufweisenden Behältern (16, 25, 26) abgehen und über ein Übergangsstück (27) mit einer gemeinsamen Patientenleitung (23) verbunden sind, mit Ventilen (5, 6, 7, 8, 11), die in jeder Förderleitung vorgesehen sind, mit einem den Durchfluß detektierenden Durchflußelement (14) und mit einer Steuereinheit (17), die mit den Ventilen, der Pumpe und dem Durchflußelement signalmäßig verbunden ist, dadurch gekennzeichnet, daß zur kontinuierlichen gleichzeitigen Infusion von Lösungen mit mindestens einer Pumpe (1 bis 4) und mindestens einer Schwerkraft-Infusionseinrichtung (16) das Durchflußelement (14) stromab der Pumpe (1 bis 4) und der Schwerkraft-Infusionseinrichtung angeordnet ist, und daß die Steuereinheit (17) die Vorrichtung bei Erkennen eines Fluidstoppes durch das Durchflußelement (14) durch Schließen der Ventile (5 bis 8, 11) und Abschalten der Pumpe desaktiviert.

2. Vorrichtung nach Anspruch 1, dadurch gekennzeichnet, daß das Durchflußelement (14) ein Tropfendetektor ist.

3. Vorrichtung nach Anspruch 1 oder 2, dadurch gekennzeichnet, daß das Durchflußelement (14) in der Patientenleitung (23) angeordnet ist.

4. Vorrichtung nach einem der Ansprüche 1 bis 3, dadurch gekennzeichnet, daß an das Übergangsstück (27) eine Entlüftungseinrichtung (22) angeschlossen ist.

5. Vorrichtung nach Anspruch 4, dadurch gekennzeichnet, daß die Entlüftungseinrichtung (22) als Stutzen ausgebildet ist, an dem ein Ventil (9) angreift.

6. Vorrichtung nach Anspruch 4, dadurch gekennzeichnet, daß die Entlüftungseinrichtung (22) mit einem hydrophoben Sterilfilter (28) abgeschlossen ist.

7. Vorrichtung nach einem der Ansprüche 3 bis 6, dadurch gekennzeichnet, daß in der Patientenleitung (23) stromauf des Durchflußelements (14) ein Luftdetektor (13) angeordnet ist, der mit der Steuereinheit (17) verbunden ist, die auf das Signal des Luftdetektors (13) in der Entlüftungsphase die Ventile (5, 8, 11, 12 und 9), die Pumpe (1 - 4) entsprechend einem vorbestimmten Programm steuert.

8. Vorrichtung nach einem der Ansprüche 1 bis 7, dadurch gekennzeichnet, daß stromab des Durchflußelements (14) in der Patientenleitung (23) ein weiteres Absperrventil (12) angeordnet ist, das bei Erkennen eines Flußstopps durch das Durchflußelement (14) mit Hilfe der Steuereinheit (17) die Patientenleitung (23) verschließt.

9. Vorrichtung nach einem der Ansprüche 1 bis 8, dadurch gekennzeichnet, daß die Schwerkraft-Infusionseinrichtung (16) über eine Verbindungsleitung (36), in die ein Ventil (11) eingeschaltet ist, an das Übergangsstück (27) angeschlossen ist.

10. Vorrichtung nach Anspruch 9, dadurch gekennzeichnet, daß das Durchflußelement (14) anstatt in der Patientenleitung (23) in der Verbindungsleitung (36) angeordnet ist.

11. Vorrichtung nach einem der Ansprüche 1 bis 10, dadurch gekennzeichnet, daß an das Übergangsstück (27) eine CVD-Meßeinrichtung (15) mit Hilfe der Verbindungsleitung (35) angeschlossen ist, die durch ein Ventil (10) absperrbar ist.

## Claims

1. Apparatus for dosed continuous simultaneous infusion of a plurality of infusion solutions or medicaments comprising at least one pump (1-4) delivery conduits (18, 19, 20, 21, 36) which lead from the containers (16, 25, 26) containing the infusion or medicament solutions and are connected via a junction piece (27) to a common patient conduit, valves (5, 6, 7, 8, 11) which are provided in each delivery conduit (23), a flow element (14) detecting the flow and a control unit (17) which is connected as regards signals to the valves, the pump and the flow element, characterized in that for dosed continuous simultaneous infusion of a plurality of infusion solutions or medicaments comprising at least one pump (1-4), and at least one gravity infusion means (16) the flow element (14) is arranged downstream of the pump (1-4) and the gravity infusion means and that the control unit (17) deactivates the apparatus on detection of a fluid stop by the flow element (14) by closing the valves (5-8, 11) and switching off the pump (1-4).

2. Apparatus according to claim 1, characterized in that the flow element (14) is a drip detector.

3. Apparatus according to claim 1 or 2, characterized in that the flow element (14) is arranged in the patient conduit (23).

4. Apparatus according to any one of the claims 1-3, characterized in that a vent means (22) is connected to the junction piece (27).

5. Apparatus according to claims 4, characterized in that the vent means (22) is formed as tube piece on which a valve (9) engages.

6. Apparatus according to claim 4, characterized in that the vent means (22) is closed with a hydrophobic sterile filter (28).

7. Apparatus according to any one of claims 3 to 6, characterized in that in the patient conduit (23) upstream of the flow element (14) an air detetor (13) is arranged which is connected to the control unit (17) which in response to the signal of the air detector (13) in the vent phase controls the valves (5, 8, 11, 12 and 9) and the pump (1-4) in accordance with a predetermined program.

8. Apparatus according to any one of claims 1 to 7, characterized in that downstream of the flow element (14) in the patient conduit (23) a further shutoff valve (12) is arranged which on detection of a fluid stop by the flow element (14) closes the patient conduit (23) with the aid of the control unit (17).

9. Apparatus according to any one of claims 1 to 8, characterized in that to the junction piece (27) a gravity infusion means (16) is connected via a connecting conduit (36) into which a valve (11) is inserted.

10. Apparatus according to claim 9, characterized in that the flow element (14) is arranged in the connecting conduit (36) and not in the patient conduit (23).

11. Apparatus according to any one of the claims 1 to 10, characterized in that to the junction piece (27) a CVP measuring means (15) is connected with the aid of a connecting conduit (35) which can be shut off by a valve (10).

## Revendications

1. Appareil permettant d'injecter, d'une manière dosée et simultanée, plusieurs solutions de perfusion ou médicaments, comprenant au moins une pompe (1 à 4), des tuyaux de circulation (18, 19, 20, 21, 36), qui partent des réservoirs (16, 25, 26) contenant les solutions de perfusion ou médicamenteuses et qui sont raccordés par un tronçon de transfert (27) à un tuyau commun de patient (23), des soupapes (5, 6, 7, 8, 11), qui sont prévues dans chaque tuyau de circulation, un organe de débit (14), détectant le débit, et une unité de commande (17) qui est reliée aux soupapes, à la pompe et à l'organe de débit d'une façon permettant la transmission de signaux, caractérisé en ce que, pour l'injection simultanée continue de solutions au moyen d'au moins une pompe (1 à 4) et d'au moins un dispositif de perfusion par gravité (16), l'organe de débit (14) est disposé en aval des pompes (1 à 4) et du dispositif de perfusion par gravité et en ce que, lorsqu'un arrêt du fluide est constaté par l'organe de débit (14), l'unité de commande (17) désactive l'appareil par fermeture des soupapes (5 à 8, 11) et mise de la pompe à l'arrêt.

2. Appareil suivant la revendication 1, caractérisé en ce que l'organe de débit (14) est un détecteur de gouttes.

3. Appareil suivant l'une des revendications 1 et 2, caractérisé en ce que l'organe de débit (14) est disposé dans le tuyau de patient (23).

4. Appareil suivant l'une des revendications 1 à 3, caractérisé en ce qu'un dispositif de mise à l'air (22) est raccordé au tronçon de transfert (27).

5. Appareil suivant la revendication 4, caractérisé en ce que le dispositif de mise à l'air (22) est réalisé sous la forme d'un embout tubulaire sur lequel agit une soupape (9).

6. Appareil suivant la revendication 4, caractérisé en ce que le dispositif de mise à l'air (22) est obturé au moyen d'un filtre stérile hydrophobe (28).

7. Appareil suivant l'une des revendications 3 à 6, caractérisé en ce que, dans le tuyau de patient (23) et en amont de l'organe de débit (14), il est disposé un détecteur d'air (13) qui est relié à l'unité de commande (17), laquelle, sur un signal du détecteur d'air (13) dans la phase de mise à l'air, commande les soupapes (5, 8, 11, 12 et 9) et la pompe (1-4) en conformité avec un programme préfixé.

8. Appareil suivant l'une des revendications 1 à 7, caractérisé en ce que, dans le tuyau de patient (23) et en aval de l'organe de débit (14), il est disposé une autre soupape de blocage (12) qui, au moyen de l'unité de commande (17), ferme le tuyau de patient (23) lorsqu'un arrêt du fluide est constaté par l'organe de débit (14).

9. Appareil suivant l'une des revendications 1 à 8, caractérisé en ce que le dispositif d'injection par gravité (16) est raccordé au tronçon de transfert (27) par un tuyau de liaison (36) dans lequel une soupape (11) est insérée.

10. Appareil suivant la revendication 9, caractérisé en ce que l'organe de débit (14) est disposé, non pas dans le tuyau de patient (23), mais dans le tuyau de liaison (36).

11. Appareil suivant l'une des revendications 1 à 10, caractérisé en ce qu'un dispositif de mesure-CVD (15) est raccordé au tronçon de transfert (27) au moyen du tuyau de liaison (35) qui peut être bloqué au moyen d'une soupape (10).
